# EUROPEAN PATENT APPLICATION

(11) **EP 0 750 899 A2**
(43) Date of publication of application: **02.01.1997**
(21) Application number: 96110394.2
(22) Date of filing: 27.06.1996
(51) Int. Cl.: A61K 7/00, C08F 20/06

(54) **An emulsifier or solubilizer which consists of a water soluble amphiphilic polyelectrolyte, and an emulsified composition or a solubilized composition and an emulsified cosmetic or a solubilized cosmetic containing it**

(30) Priority: 30.06.1995 JP 188164/95; 26.07.1995 JP 209907/95; 15.11.1995 JP 322226/95; 15.11.1995 JP 322227/95; 15.11.1995 JP 322228/95; 15.11.1995 JP 322229/95; 15.11.1995 JP 322230/95
(71) Applicant: SHISEIDO COMPANY LIMITED, Tokyo 104-10 (JP)
(72) Inventor: Kobayashi, Atsushi, c/o Shiseido Research Center, Yokohama-shi, Kanagawa 233 (JP); Matsuzaki, Fumiaki, c/o Shiseido Research Center, Yokohama-shi, Kanagawa 233 (JP); Yanaki, Toshio, c/o Shiseido Research Center, Yokohama-shi, Kanagawa 233 (JP); Yamaguchi, Michihiro, c/o Shiseido Research Center, Yokohama-shi, Kanagawa 233 (JP)
(74) Representative: TER MEER - MÜLLER - STEINMEISTER & PARTNER

(57) **Abstract**

An emulsified composition and emulsified cosmetic or a solubilized composition and solubilized cosmetic with good stability and usability can be provided by using an emulsifier or a solubilizer consisting of a water soluble amphiphilic polyelectrolyte obtained by copolymerizing (meth)acrylamido alkylsulfonic acid and N-substituted (meth)acrylamide.

Also, an emulsified composition and emulsified cosmetic or a solubilized composition and solubilized cosmetic with good stability and usability can be provided by using an emulsifier or a solubilizer consisting of a water soluble cross-linked amphiphilic polyelectrolyte obtained by copolymerizing (meth) acrylamido alkylsulfonic acid, N-substituted (meth) acrylamide and a cross-linking agent.

Furthermore, an emulsified composition and emulsified cosmetic or a solubilized composition and solubilized cosmetic with good stability and usability can be provided by using an emulsifier or a solubilizer consisting of a water soluble amphiphilic polyelectrolyte obtained by copolymerizing (meth) acrylamido alkylsulfonic acid and (meth)acrylate.

Also, an emulsified composition and emulsified cosmetic or a solubilized composition and solubilized cosmetic with good stability and usability can be provided by using an emulsifier or a solubilizer consisting of a water soluble cross-linked amphiphilic polyelectrolyte obtained by copolymerizing (meth) acrylamido alkylsulfonic acid (meth)acrylate and a cross-linking agent.

## Description

### Background of the Invention

### 1. Field of the Invention

The present invention relates to an emulsifier which consists of a water soluble amphiphilic polyelectrolyte or a water soluble cross-linked amphiphilic polyelectrolyte.

Also, the present invention relates to a solubilizer which consists of a water soluble amphiphilic polyelectrolyte or a water soluble cross-linked amphiphilic polyelectrolyte.

Furthermore, the present invention relates to an emulsified composition and emulsified cosmetic containing the aforementioned emulsifier.

Also, the present invention relates to a solubilized composition and solubilized cosmetic containing the aforementioned solubilizer.

### 2. The prior Art

Conventionally, a great deal of research has been conducted on emulsification, and many emulsifiers have been developed. The progress of emulsification technology has also been remarkable, and very stable emulsions have been widely used in every industry.

However, most of them employ for the emulsifier a nonionic surfactant containing polyoxyethylene chains, an anionic surfactant represented by aliphatic soap or an ampholytic surfactant, and many people, particularly general consumers, are concerned about safety.

Therefore, in recent years, a method which obtains an emulsified composition by using an alkylated water soluble polymer has been investigated. Examples include alkylated carboxy vinyl polymers known by their product names such as Carbopol 1342, Pemulen TR-1 and Pemulen TR-2 (from BF Goodrich).

However, these polymers have relatively low emulsification power and poor emulsification stability. Therefore, in order to improve stability, it is necessary to add a large amount. However, the addition of such a large amount causes elasticization (gelation) and significantly reduces the usability of the emulsion.

The inventors conducted earnest research to solve the aforementioned problem and discovered that a water soluble amphiphilic polyelectrolyte obtained by copolymerizing (meth)acrylamide alkylsulfonic acid and N-substituted (meth)acrylamide or (meth) acrylate and a water soluble cross-linked amphiphilic polyelectrolyte prepared by cross-linking the former could be an emulsifier with which an emulsified composition with good emulsification stability, safety and usability could be prepared, thus completing the present invention.

As for the solubilizer, a great deal of research has been conducted on solubilization, and many solubilizers have been developed. Very stable solubilized systems have been widely used in every industry.

However, most of them employ for the solubilizer a nonionic surfactant containing polyoxyethylene chains such as polyoxyethylene stearate derivatives, polyoxyethylene oleyl ether, polyoxyethylene sorbitan oleate and polyoxyethylene hydrogenated castor oil, and many people, particularly general consumers, are concerned about safety.

Therefore, in recent years, a method which uses a polymer surfactant, which is expected to be safer than conventional surfactants, has been investigated. However, the solubilization process tends to be complex and none is commercially available which can be manufactured easily at a low cost.

The inventors conducted earnest research to solve the aforementioned problem and discovered that a water soluble amphiphilic polyelectrolyte obtained by copolymerizing (meth)acrylamide alkylsulfonic acid and N-substituted (meth)acrylamide or (meth) acrylate and a water soluble cross-linked amphiphilic polyelectrolyte prepared by cross-linking the former could be a solubilizer with which a solubilized composition with good safety and usability could be easily manufactured, thus completing the present invention.

### Brief Summary of the Invention

That is, the present invention provides an emulsifier which consists of a water soluble amphiphilic polyelectrolyte obtained by copolymerizing (meth) acrylamide alkylsulfonic acid and/or its salt and N-substituted (meth)acrylamide, or by neutralizing the obtained copolymer with an alkali agent.

Also, the present invention provides an emulsifier which consists of a water soluble cross-linked amphiphilic polyelectrolyte obtained by copolymerizing (meth) acrylamide alkylsulfonic acid and/or its salt, N-substituted (meth)acrylamide and a cross-linking agent, or by neutralizing the obtained cross-linked copolymer with an alkali agent.

Also, the present invention provides an emulsifier which consists of a water soluble amphiphilic polyelectrolyte obtained by copolymerizing (meth)acrylamide alkylsulfonic acid and/or its salt and (meth)acrylate, or by neutralizing the obtained copolymer with an alkali agent.

Also, the present invention provides an emulsifier which consists of a water soluble cross-linked amphiphilic polyelectrolyte obtained by copolymerizing (meth) acrylamide alkylsulfonic acid and/or its salt, (meth) acrylate and a cross-linking agent, or by neutralizing the obtained cross-linked copolymer with an alkali agent.

Also, the present invention provides an emulsified composition and an emulsified cosmetic comprising the aforementioned emulsifier(s).

Also, the present invention provides a solubilizer which consists of a water soluble amphiphilic polyelectrolyte obtained by copolymerizing (meth)acrylamide alkylsulfonic acid and/or its salt and N-substituted (meth)acrylamide, or by neutralizing the obtained copolymer with an alkali agent.

Also, the present invention provides a solubilizer which consists of a water soluble cross-linked amphiphilic polyelectrolyte obtained by copolymerizing (meth) acrylamide alkylsulfonic acid and/or its salt, N-substituted (meth)acrylamide and a cross-linking agent, or by neutralizing the obtained cross-linked copolymer with an alkali agent.

Also, the present invention provides a solubilizer which consists of a water soluble amphiphilic polyelectrolyte obtained by copolymerizing (meth)acrylamide alkylsulfonic acid and/or its salt and (meth)acrylate, or by neutralizing the obtained copolymer with an alkali agent.

Also, the present invention provides a solubilizer which consists of a water soluble cross-linked amphiphilic polyelectrolyte obtained by copolymerizing (meth) acrylamide alkylsulfonic acid and/or its salt, (meth) acrylate and a cross-linking agent, or by neutralizing the obtained cross-linked copolymer with an alkali agent.

Also, the present invention provides a solubilized composition and a solubilized cosmetic comprising the aforementioned solubilizer(s).

### Detailed Description

The configuration of the present invention is described in detail below.

The water soluble amphiphilic polyelectrolyte used in the present invention is a copolymer obtained by copolymerizing (meth)acrylamide alkylsulfonic acid and/or its salts and N-substituted (meth)acrylamide. For (meth)acrylamide alkylsulfonic acid, its salts can be used, too. The salts can be used independently or in combination with (meth)acrylamide alkylsulfonic acid for copolymerization. Examples of the salts of (meth)acrylamide alkylsulfonic acid which can be used include salts with alkali metal, ammonia and organic amine such as triethylamine and triethanol amine. It is also possible to obtain the water soluble amphiphilic polyelectrolyte of the present invention by using an alkali agent to neutralize the (meth) acrylamide alkylsulfonic acid units of the obtained copolymer.

The water soluble amphiphilic polyelectrolyte thus obtained characteristically contains (meth)acrylamide alkylsulfonic acid units (copolymerization units originated from (meth)acrylamide alkylsulfonic acid and/or its salts) which give it hydrophilicity and N-substituted (meth)acrylamide units (copolymerization units originated from N-substituted (meth)acrylamide) which give it hydrophobicity, and functions as a water soluble emulsifier or solubilizer with these ingredients and a certain ingredient composition.

The ingredient composition of the water soluble cross-linked amphiphilic polyelectrolyte containing these ingredients is within the range in which the obtained copolymer is water soluble and also within the range in which it functions as an emulsifier or a solubilizer. That is, the (meth)acrylamide alkylsulfonic acid units account for 15 wt% or more to less than 60 wt%, preferably 20 wt% or more to less than 50 wt % of the total polymer. The N-substituted (meth) acrylamide units account for 40 wt% or more to less than 85 wt%, preferably 50 wt% or more to less than 80 wt% of the total polymer.

Also, the water soluble cross-linked amphiphilic polyelectrolyte used in the present invention is a cross-linked copolymer obtained by copolymerizing (meth)acrylamide alkylsulfonic acid and/or its salts, N-substituted (meth)acrylamide and a cross-linking agent. For (meth)acrylamide alkylsulfonic acid, its salts can be used, too. The salts can be used independently or in combination with (meth)acrylamide alkylsulfonic acid for copolymerization. Examples of the salts of (meth)acrylamide alkylsulfonic acid which can be used include salts with alkali metal, ammonia and organic amine such as triethylamine and triethanol amine. It is also possible to obtain the water soluble cross-linked amphiphilic polyelectrolyte of the present invention by using an alkali agent to neutralize the (meth)acrylamide alkylsulfonic acid units of the obtained copolymer.

The water soluble cross-linked amphiphilic polyelectrolyte thus obtained characteristically contains (meth)acrylamide alkylsulfonic acid units (copolymerization units originated from (meth)acrylamide alkylsulfonic acid and)or its salts) which give it hydrophilicity and N-substituted (meth)acrylamide units (copolymerization units originated from N-substituted (meth)acrylamide) which give it hydrophobicity, and functions as a water soluble emulsifier or solubilizer with these ingredients and a certain ingredient composition.

The ingredient composition of the water soluble cross-linked amphiphilic polyelectrolyte containing these ingredients is within the range in which the obtained copolymer is water soluble and also within the range in which it functions as an emulsifier or a solubilizer. That is, the (meth)acrylamide alkylsulfonic acid units account for 15 wt% or more to less than 60 wt%, preferably 20 wt% or more to less than 50 wt% of the total polymer. The N-substituted (meth) acrylamide units account for 40 wt% or more to less than 85 wt%, preferably 50 wt% or more to less than 80 wt% of the total polymer. The cross-linking agent units account for 0.001 wt% or more to 2 wt% or less of the total of the copolymer.

Another water soluble amphiphilic polymer electrolyte used in the present invention is a copolymer obtained by copolymerizing (meth)acrylamide alkylsulfonic acid and/or its salts and (meth)acrylate. For (meth) acrylamide alkylsulfonic acid, its salts can be used, too. The salts can be used independently or in combination with (meth)acrylamide alkylsulfonic acid for copolymerization. Examples of the salts of (meth)acrylamide alkylsulfonic acid which can be used include salts with alkali metal, ammonia and organic amine such as triethylamine and triethanol amine. It is also possible to obtain the water soluble amphiphilic polyelectrolyte of the present invention by using an alkali agent to neutralize the (meth)acrylamide alkylsulfonic acid units of the obtained copolymer.

The water soluble amphiphilic polyelectrolyte thus obtained characteristically contains (meth) acrylamide alkylsulfonic acid units (copolymerization units originated from (meth)acrylamide alkylsulfonic acid and/or its salts) which give it hydrophilicity and (meth)acrylate units (copolymerization units originated from (meth)acrylate) which give it hydrophobicity, and functions as a water soluble emulsifier or solubilizer with these ingredients and a certain ingredient composition.

The ingredient composition of the water soluble amphiphilic polyelectrolyte containing these ingredients is within the range in which the obtained copolymer is water soluble and also within the range in which it functions as an emulsifier or a solubilizer. That is, the (meth)acrylamide alkylsulfonic acid units account for 60 wt% or more to less than 99 wt%, preferably 65 wt% or more to less than 95 wt % of the total polymer. The (meth)acrylate units account for 1 wt% or more to less than 40 wt%, preferably 5 wt% or more to less than 35 wt% of the total polymer.

Also, another water soluble cross-linked amphiphilic polyelectrolyte used in the present invention is a cross-linked copolymer obtained by copolymerizing (meth)acrylamide alkylsulfonic acid and/or its salts, (meth)acrylate and a cross-linking agent. For (meth) acrylamide alkylsulfonic acid, its salts can be used, too. The salts can be used independently or in combination with (meth)acrylamide alkylsulfonic acid for copolymerization. Examples of the salts of (meth)acrylamide alkylsulfonic acid which can be used include salts with alkali metal, ammonia and organic amine such as triethylamine and triethanol amine. It is also possible to obtain the water soluble cross-linked amphiphilic polyelectrolyte of the present invention by using an alkali agent to neutralize the (meth)acrylamide alkylsulfonic acid units of the obtained copolymer.

The water soluble cross-linked amphiphilic polyelectrolyte thus obtained characteristically contains (meth)acrylamide alkylsulfonic acid units (copolymerization units originated from (meth)acrylamide alkylsulfonic acid and/or its salts) which give it hydrophilicity and (meth)acrylate units (copolymerization units originated from (meth)acrylate) which give it hydrophobicity, and functions as a water soluble emulsifier or solubilizer with these ingredients and a certain ingredient composition.

The ingredient composition of the water soluble cross-linked amphiphilic polyelectrolyte containing these ingredients is within the range in which the obtained copolymer is water soluble and also within the range in which it functions as an emulsifier or a solubilizer. That is, the (meth)acrylamide alkylsulfonic acid units account for 60 wt% or more to less than 99 wt%, preferably 65 wt% or more to less than 95 wt % of the total polymer. The (meth)acrylate units account for 1 wt% or more to less than 40 wt%, preferably 5 wt% or more to less than 35 wt% of the total polymer. The cross-linking agent units account for 0.001 wt % or more to 2 wt% or less of the total of the copolymer.

Examples of the aforementioned (meth)acrylamide alkylsulfonic acid include acrylamido methanesulfonic acid, acrylamido ethanesulfonic acid, acrylamido propanesulfonic acid, 2-acrylamido-2-methylpropanesulfonic acid, 2-methacrylamido-2-methylpropanesulfonic acid and 2-acrylamido-n-butanesulfonic acid.

Selection of the N-substituted (meth)acrylamide is not limited in particular, and examples include cyclohexyl acrylamide, cyclohexyl methacrylamide, lauryl acrylamide, lauryl methacrylamide, phenyl acrylamide, phenyl methacrylamide, 2-naphtyl acrylamide, 2-naphtyl methacrylamide, N-n-dodecyl-N-methyl acrylamide and N-n-dodecyl-N-methyl methacrylamide.

Selection of the (meth)acrylate is not limited in particular, and examples include butyl acrylate, butyl methacrylate, octyl acrylate, octyl methacrylate, 2-ethylhexyl acrylate, 2-ethylhexyl methacrylate, decyl acrylate, decyl methacrylate, lauryl acrylate, lauryl methacrylate, stearyl acrylate and stearyl methacrylate.

The cross-linking agent is a monomer containing 2 or more polymerizing double bonds in the molecule. Examples include methylenebisacrylamide, ethylenebisacrylamide, ethylene dimethacrylate, ethylene diacrylate, divinylbenzene and trimethylolpropane trimethacrylate.

For the polymerization method of the copolymer, prior art polymerization methods such as the solution polymerization method, bulk polymerization method and precipitation polymerization method can be used.

Selection of the polymerization initiator is not limited in particular as long as it has the ability to start radical polymerization. Examples include benzoyl peroxide, azobisisobutyronitrile, potassium persulfate and ammonium persulfate.

The copolymer-type water soluble amphiphilic polyelectrolyte or water soluble cross-linked amphiphilic polyelectrolyte obtained by the aforementioned method (s) may contain other vinyl monomers in addition to the (meth)acrylamide alkylsulfonic acid units, N-substituted (meth)acrylamide units or (meth)acrylate units and the cross-linking units, as long as the effect of the present invention, i.e. providing a water soluble emulsifier or solubilizer, is not jeopardized.

The emulsified composition and emulsified cosmetic of the present invention are obtained by blending in the emulsifier consisting of the water soluble amphiphilic polyelectrolyte or water soluble cross-linked amphiphilic polyelectrolyte of the present invention. The blend ratio is 0.01-30 wt%, preferably 0.1-20 wt%. If it is less than 0.01 wt%, then emulsification is not sufficient. If it is more than 30 wt% then gelation occurs.

Also, the solubilized composition and solubilized cosmetic of the present invention are obtained by blending in the solubilizer consisting of the water soluble amphiphilic polyelectrolyte or water soluble cross-linked amphiphilic polyelectrolyte of the present invention. The blend ratio is 0.001-30 wt%, preferably 0.01-20 wt%. If it is less than 0.001 wt%, then solubilization is not sufficient. If it is more than 30 wt% then gelation occurs.

The solubilizer consisting of the water soluble amphiphilic polyelectrolyte of the present invention is preferably used as a solubilizer to solubilize perfume and drugs in particular.

Ingredients typically added to an emulsified composition and an emulsified cosmetic or a solubilized composition and a solubilized cosmetic, including oil, surfactants, humectants, ultraviolet light absorbents, chelating agents, pH control agents, preservatives, thickeners, dyes, pigments, perfumes, colorings, plasticizers and organic solvents may be added to the emulsified composition and the emulsified cosmetic or the solubilized composition and the solubilized cosmetic of the present invention as long as the effects of the present invention are not jeopardized.

According to the present invention, an emulsified composition and emulsified cosmetic or a solubilized composition and solubilized cosmetic with good stability and usability can be provided by using an emulsifier or a solubilizer consisting of a water soluble amphiphilic polyelectrolyte obtained by copolymerizing (meth) acrylamide alkylsulfonic acid and N-substituted (meth) acrylamide.

Also, according to the present invention, an emulsified composition and emulsified cosmetic or a solubilized composition and solubilized cosmetic with good stability and usability can be provided by using an emulsifier or a solubilizer consisting of a water soluble cross-linked amphiphilic polyelectrolyte obtained by copolymerizing (meth)acrylamide alkylsulfonic acid, N-substituted (meth)acrylamide and a cross-linking agent.

Furthermore, according to the present invention, an emulsified composition and emulsified cosmetic or a solubilized composition and solubilized cosmetic with good stability and usability can be provided by using an emulsifier or a solubilizer consisting of a water soluble amphiphilic polyelectrolyte obtained by copolymerizing (meth)acrylamide alkylsulfonic acid and (meth)acrylate.

Also, according to the present invention, an emulsified composition and emulsified cosmetic or a solubilized composition and solubilized cosmetic with good stability and usability can be provided by using an emulsifier or a solubilizer consisting of a water soluble cross-linked amphiphilic polyelectrolyte obtained by copolymerizing (meth)acrylamide alkylsulfonic acid, (meth)acrylate and a cross-linking agent.

### Examples

Synthesis examples, embodiment examples and comparative examples of the water soluble amphiphilic polyelectrolyte and the water soluble cross-linked amphiphilic polyelectrolyte used in the present invention are listed below to describe further details of the present invention. The present invention is not limited to these embodiment examples.

### [Synthesis example 1]

25.9 weight parts of 2-acrylamido-2-methylpropanesulfonic acid, 74.0 weight parts of lauryl methacrylamide, 0.1 weight parts of azobisisobutyronitrile and 100 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 20 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble amphiphilic polyelectrolyte.

### [Synthesis example 2]

28.9 weight parts of 2-acrylamido-2-methylpropanesulfonic acid, 70.9 weight parts of t-butylacrylamide, 0.2 weight parts of azobisisobutyronitrile and 260 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 40 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble amphiphilic polyelectrolyte.

### [Synthesis example 3]

53.5 weight parts of 2-acrylamido-2-methylpropanesulfonic acid, 46.3 weight parts of adamantylmethacrylamide, 0.2 weight parts of azobisisobutyronitrile and 150 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 30 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble amphiphilic polyelectrolyte.

### [Synthesis example 4]

25.9 weight parts of 2-acrylamido-2-methylpropanesulfonic acid, 73.9 weight parts of lauryl methacrylamide, 0.18 weight parts of azobisisobutyronitrile, 0.02 weight parts of methylenebisacrylamide and 100 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 20 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble cross-linked amphiphilic polyelectrolyte.

### [Synthesis example 5]

28.7 weight parts of 2-acrylamido-2-methylpropanesulfonic acid, 71.0 weight parts of t-butyl acrylamide, 0.25 weight parts of azobisisobutyronitrile, 0.05 weight parts of methylenebisacrylamide and 260 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 40 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble cross-linked amphiphilic polyelectrolyte.

### [Synthesis example 6]

53.45 weight parts of 2-acrylamido-2-methylpropanesulfonic acid, 46.25 weight parts of adamantyl methacrylamide, 0.15 weight parts of azobisisobutyronitrile, 0.15 weight parts of methylenebisacrylamide and 150 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 30 hours. After the product solution was taken out of the reaction vessel, re -precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble cross-linked amphiphilic polyelectrolyte.

### [Synthesis example 7]

66.70 weight parts of 2-acrylamido-2-methylpropanesulfonic acid, 33.15 weight parts of lauryl acrylamide, 0.15 weight parts of azobisisobutyronitrile and 100 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 20 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble amphiphilic polyelectrolyte.

### [Synthesis example 8]

70.75 weight parts of 2-acrylamido-2-methylpropanesulfonic acid, 29.00 weight parts of 2-ethylhexyl methacrylamide, 0.25 weight parts of azobisisobutyronitrile and 260 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 40 hours. After the product solution was taken out of the reaction vessel, re -precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble amphiphilic polyelectrolyte.

### [Synthesis example 9]

96.04 weight parts of 2-acrylamido-2-methylpropanesulfonic acid, 3.65 weight parts of lauryl methacrylate, 0.31 weight parts of azobisisobutyronitrile and 150 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 30 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble amphiphilic polyelectrolyte.

### [Synthesis example 10]

66.65 weight parts of 2-acrylamido-2-methylpropanesulfonic acid, 33.13 weight parts of lauryl acrylate, 0.15 weight parts of azobisisobutyronitrile, 0.07 weight parts of methylenebisacrylamide and 100 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 20 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble cross-linked amphiphilic polyelectrolyte.

### [Synthesis example 11]

70.64 weight parts of 2-acrylamido-2-methylpropanesulfonic acid, 28.96 weight parts of 2-ethylhexyl methacrylate, 0.24 weight parts of azobisisobutyronitrile, 0.16 weight parts of methylenebisacrylamide and 260 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 40 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble cross-linked amphiphilic polyelectrolyte.

### [Synthesis example 12]

95.69 weight parts of 2-acrylamido-2-methylpropanesulfonic acid, 3.63 weight parts of lauryl methacrylate, 0.31 weight parts of azobisisobutyronitrile, 0.37 weight parts of methylenebisacrylamide and 150 weight parts of N,N-dimethylformamide were fed into a reaction vessel equipped with a stirrer, a reflux condenser and nitrogen feeding tubes, and 2 hours of stirring was conducted under nitrogen gas flow at room temperature. The temperature of the reaction system was raised to 60°C and the reaction was carried out for 30 hours. After the product solution was taken out of the reaction vessel, re-precipitation using ether was conducted 3 times to obtain a polymer powder. This powder was dissolved in an aqueous solution of NaOH and dialysis was conducted for approximately 7 days. The aqueous solution was then condensed and lyophilized to obtain the water soluble cross-linked amphiphilic polyelectrolyte.

### [Embodiment examples 1-3 and comparative examples 1-3]

Using the water soluble amphiphilic polyelectrolyte synthesized in the aforementioned synthesis example 1, synthesis example 4, synthesis example 7 or synthesis example 10 for the emulsifier, emulsified compositions of embodiment examples 1-3 and comparative examples 1-3 were prepared with the recipes shown in Table 1. Stability after letting them stand for 1 month at 50°C and at 0°C was evaluated. Stability was evaluated using the following three step rating.

### 50°C

- ○ :: No oil separation is observed.
- △ :: Slight oil separation is observed.
- X :: Oil separation is observed.

### 0°C

- ○ :: No gelation is observed and the sample is easily taken.
- △ :: Slight gelation is observed, but the sample is easily taken.
- X :: Gelation is observed, and the sample is not easily taken.

**Table 1**

| | Embodiment example 1 | Emebodiment example 2 | Embodiment example 3 | Comparative example 1 | Comparative example 2 | Comparative example 3 |
|---|---|---|---|---|---|---|
| Liquid paraffin | 20.0% | 20.0% | 20.0% | 20.0% | 20.0% | 20.0% |
| Synthesis examples 1, 4, 7 or 10 | 0.2 | 2.0 | 0.2 | - | - | - |
| PEMULEN TR - 1 | - | - | - | 0.2 | 2.0 | - |
| Carboxy vinyl polymer (trademark : Hiviswako 105) | - | - | 0.2 | - | - | 2.0 |
| Triethanol amine | - | - | 0.4 | 0.4 | 4.0 | 4.0 |
| Glycerine | 10.0 | 10.0 | 10.0 | 10.0 | 10.0 | 10.0 |
| Trisodium edetate | 0.1 | 0.1 | 0.1 | 0.1 | 0.1 | 0.1 |
| Purified water | to 100 | to 100 | to 100 | to 100 | to 100 | to 100 |
| 50°C after 1 month | △ | ○ | ○ | X | ○ | X |
| 0°C after 1 month | ○ | ○ | ○ | ○ | X | X |

The results listed in Table 1 show that emulsified compositions with good stability are obtained in embodiment examples 1-3. Comparative examples 1 and 2 indicate that the conventional alkylated carboxy vinyl polymer(s) allows oil separation when it is used in small amounts and that, when it is used in large amounts, although oil separation can be prevented, elasticization occurs. Comparative example 3 indicates that the carboxy vinyl polymer (trade name: Hiviswako 105) does not have emulsification power.

### [Embodiment example 4] Emulsion

| (Recipe) | wt% |
|---|---|
| Glycerine | 5.0 |
| Sorbitol | 5.0 |
| Emulsifier obtained in synthesis example 1, emulsifier obtained in synthesis example 4, emulsifier obtained in synthesis example 7 or emulsifier obtained in synthesis example 10 | 3.0 |
| Sodium hexamethaphosphate | 0.01 |
| Vaseline | 0.2 |
| Liquid paraffin | 5.0 |
| Glyceryl diisostearate | 10.0 |
| Cetyl octanoate | 2.0 |
| Perfume | 0.01 |
| Preservative | 0.1 |
| Purified water | Balance |

The emulsion, which is an emulsified cosmetic of the present invention prepared according to a conventional preparation method, was stable after having been let stand for 1 month at 50°C and at 0°C. Usability was good as well.

### [Embodiment example 5] Cream

| (Recipe) | wt% |
|---|---|
| Vaseline | 10.0 |
| Liquid paraffin | 10.0 |
| Cetanol | 2.0 |
| Microcrystalline wax | 2.0 |
| Methylpolysiloxane | 5.0 |
| Jojoba oil | 5.0 |
| Emulsifier obtained in synthesis example 2, emulsifier obtained in synthesis example 5, emulsifier obtained in synthesis example 8 or emulsifier obtained in synthesis example 11 | 5.0 |
| Propylene glycol | 5.0 |
| Glycerine | 5.0 |
| Perfume | 0.05 |
| Preservative | 0.1 |
| Purified water | Balance |

The cream, which is an emulsified cosmetic of the present invention prepared according to a conventional preparation method, was stable after having been let stand for 1 month at 50°C and at 5°C. Usability was good as well.

Embodiment examples for the solubilizer, solubilized composition and solubilized cosmetic of the present invention are shown below. Their performance was measured using the following methods and the results are shown in Table 2.

[Solubilization power] 0.1 g of the sample, 0.1 g of the preservative and the test weight of an oil soluble drug (vitamin E acetate) were homogeneously dissolved in 10 g of ethanol, and purified water was added to obtain a total amount of 100 g. The blend ratio of the oil soluble drug which makes the transmittance of the system 80% at 700 nm was evaluated using the following four step rating.
- ⓞ :: The weight is equal to or greater than the sample weight.
- ○ :: The weight is equal to or greater than half and less than the sample weight.
- △ :: The weight is equal to or greater than a third and less than a half of the sample weight.
- X :: The weight is less than a third of the sample weight.

[Haziness] The samples in a solubilized state were evaluated after having been let stand for four weeks at 50°C for haziness using the following three step rating. Haziness stands for a state in which the system has become slightly nebulous and developed a low level of mist and/or sedimentation.
- ○ :: No haziness is observed.
- △ :: Some haziness is observed.
- X :: Haziness is observed.

[Defoaming properties] 10 ml of a 1% aqueous solution was sampled in a test tube and, after 30 seconds of vigorous shaking, defoaming was evaluated using the following 3 step rating.
- ○ :: Very good
- △ :: Good
- X :: Poor

[pH reduction] The 1% aqueous solution was let stand for two months at 50°C, and reduction in pH was evaluated using the following three step rating.
- ○ :: pH reduction is less than 1.
- △ :: pH reduction is equal to or greater than 1 and less than 2.
- X :: pH reduction is 2 or more.

**Table 2**

| Sample | | Solubilization power | Haziness | Defoaming properties | pH reduction |
|---|---|---|---|---|---|
| Solubilizers of the present invention | Synthesis example 1, 4, 7, 8, 10 or 11 | ○ | ○ | ○ | ○ |
| | Synthesis example 2 or 5 | △ | ○ | ○ | ○ |
| Conventional solubilizers | Polyoxyethylene (30) hydrogenated castor oil | ⓞ | △ | X | X |
| | Polyoxyethylene (20) oleyl ether | X | X | X | △ |
| | Polyoxyethylene (35) polyoxypropylene glycol (40) | X | X | △ | △ |

The results listed in Table 2 show that the solubilizers of the present invention have superior performance. The conventional polyoxyethylene (30) hydrogenated castor oil has a high solubilization power, but is inferior to the solubilizers of the present invention in terms of defoaming properties and pH reduction. Polyoxyethylene (20) oleyl ether and polyoxyethylene (35) polyoxypropylene glycol (40) are inferior to the solubilizers of the present invention in terms of all items of the performance testing.

### [Embodiment example 6] Essence

| (Recipe) | wt% |
|---|---|
| Glycerine | 4.0 |
| 1,3-butylene glycol | 6.0 |
| Solubilize obtained in synthesis example 1 solubilizer obtained in synthesis example 4, solubilizer obtained in synthesis example 7 or solubilizer obtained in synthesis example 10 | 1.0 |
| Ethanol | 10.0 |
| Arbutin | 0.1 |
| Olive oil | 0.1 |
| Hydroxyethyl cellulose | 0.1 |
| Sequestering agent | Appropriate amount |
| Anti-discoloration agent | Appropriate amount |
| Perfume | Appropriate amount |
| Preservative | Appropriate amount |
| Purified water | Balance |

The essence, which is a solubilized cosmetic of the present invention prepared according to a conventional preparation method, was stable after having been let stand for 1 month at 50°C and at 5°C. Usability was good as well.

[Embodiment example 7] Preparation was conducted in the same manner as in embodiment example 6 except for the fact that the solubilizer obtained in synthesis example 1 was replaced by the solubilizer obtained in synthesis example 2, 5 8 or 11.

[Comparative example 4] Preparation was conducted in the same manner as in embodiment example 6 except for the fact that the solubilizer obtained in synthesis example 1 was replaced by polyoxyethylene (30) hydrogenated castor oil.

[Comparative example 5] Preparation was conducted in the same manner as in embodiment example 6 except for the fact that the solubilizer obtained in synthesis example 1 was replaced by polyoxyethylene (20) oleyl ether.

[Comparative example 6] Preparation was conducted in the same manner as in embodiment example 6 except for the fact that the solubilizer obtained in synthesis example 1 was replaced by polyoxyethylene (35) polyoxypropylene glycol (40).

Ten specialized panelists used the essences prepared in embodiment examples 6 and 7 and comparative examples 4, 5 and 6, conducted the sensory tests, and evaluated usability using the following four step rating.
- ⓞ :: 8 or more of 10 panelists gave a positive response.
- ○ :: 6-7 of 10 panelists gave a positive response.
- △ :: 4-5 of 10 panelists gave a positive response.
- X :: 3 or fewer of 10 panelists gave a positive response.

**Table 3**

| | Embodiment example 6 | Embodiment example 7 | Comparative example 4 | Comparative example 5 | Comparative example 6 |
|---|---|---|---|---|---|
| Refreshing feeling | Ⓞ | ○ | X | △ | △ |
| Smooth feeling | Ⓞ | Ⓞ | △ | X | △ |
| Dew feeling | ○ | ○ | △ | X | X |
| Total evaluation | Ⓞ | ○ | △ | X | △ |

The results listed in Table 3 show that solubilized cosmetics with good usability can be obtained by embodiment examples 6 and 7 which use the solubilizers of the present invention.

### [Embodiment example 8] Lotion

| (Recipe) | wt% |
|---|---|
| Glycerine | 6.0 |
| Sorbitol | 3.0 |
| 1,3-butylene glycol | 5.0 |
| Hyaluronic acid | 0.1 |
| Glyceryl tri-2-ethylhexanoate | 0.2 |
| Vitamin E acetate | 0.02 |
| Solubilizer obtained in synthesis example 2, solubilizer obtained in synthesis example 5, solubilizer obtained in synthesis example 8 or solubilizer obtained in synthesis example 11 | 0.2 |
| Ethanol | 2.0 |
| Glycerine | 5.0 |
| Sequestering agent | Appropriate amount |
| Anti-discoloration agent | Appropriate amount |
| Perfume | Appropriate amount |
| Preservative | Appropriate amount |
| Purified water | Balance |

The lotion, which is a solubilized cosmetic of the present invention prepared according to a conventional preparation method, was stable after having been let stand for 1 month at 50°C and at 5°C. Usability was good as well.

### [Embodiment example 9] Hair tonic

| (Recipe) | wt% |
|---|---|
| Ethanol | 66.0 |
| Solubilizer obtained in synthesis example 1, solubilizer obtained in synthesis example 4, solubilizer obtained in synthesis example 7 or solubilizer obtained in synthesis example 10 | 20.0 |
| Propylene glycol | 2.0 |
| Hinokitiol | 0.02 |
| Pyridoxine hydrochloride | 0.05 |
| Pantothenylethyl ether | 0.2 |
| 1-menthol | 0.6 |
| Vitamin E acetate | 0.2 |
| pH control agent | Appropriate amount |
| Coloring agent | Appropriate amount |
| Perfume | Appropriate amount |
| Purified water | Balance |

The hair tonic, which is a solubilized cosmetic of the present invention prepared according to a conventional preparation method, was stable after having been let stand for 1 month at 50°C and at 5°C. Usability was good as well.

## Claims

1. An emulsifier which consists of a water soluble amphiphilic polyelectrolyte obtained by copolymerizing (meth)acrylamido alkylsulfonic acid and/or its salt and N-substituted (meth)acrylamide, or by neutralizing the obtained copolymer with an alkali agent.

2. An emulsifier which consists of a water soluble cross-linked amphiphilic polyelectrolyte obtained by copolymerizing (meth)acrylamido alkylsulfonic acid and/or its salt, N-substituted (meth)acrylamide and a cross-linking agent, or by neutralizing the obtained cross-linked copolymer with an alkali agent.

3. An emulsifier which consists of a water soluble amphiphilic polyelectrolyte obtained by copolymerizing (meth)acrylamido alkylsulfonic acid and/or its salt and (meth)acrylate, or by neutralizing the obtained copolymer with an alkali agent.

4. An emulsifier which consists of a water soluble cross-linked amphiphilic polyelectrolyte obtained by copolymerizing (meth)acrylamido alkylsulfonic acid and/or its salt, (meth)acrylate and a cross-linking agent, or by neutralizing the obtained cross -linked copolymer with an alkali agent.

5. The emulsifier of claim 1 or 2 wherein at least 15 wt% of the total of said water soluble amphiphilic polyelectrolyte is (meth)acrylamido alkylsulfonic acid units.

6. The emulsifier of claim 1 or 2 wherein at least 40 wt% of the total of said water soluble amphiphilic polyelectrolyte is N-substituted (meth) acrylamide units.

7. The emulsifier of claim 3 or 4 wherein at least 60 wt% of the total of said water soluble amphiphilic polyelectrolyte is (meth)acrylamido alkylsulfonic acid units.

8. The emulsifier of claim 3 or 4 wherein at least 1 wt% of the total of said water soluble amphiphilic polyelectrolyte is (meth)acrylate units.

9. An emulsified composition comprising the emulsifier of claim 1, 2, 3 or 4.

10. The emulsified composition of claim 9 wherein the blend ratio of said emulsifier is 0.1 -20 wt%.

11. An emulsified cosmetic comprising the emulsifier of claim 1, 2, 3 or 4.

12. A solubilizer which consists of a water soluble amphiphilic polyelectrolyte obtained by copolymerizing (meth)acrylamido alkylsulfonic acid and/or its salt and N-substituted (meth)acrylamide, or by neutralizing the obtained copolymer with an alkali agent.

13. A solubilizer which consists of a water soluble cross-linked amphiphilic polyelectrolyte obtained by copolymerizing (meth)acrylamido alkylsulfonic acid and/or its salt, N-substituted (meth)acrylamide and a cross-linking agent, or by neutralizing the obtained cross-linked copolymer with an alkali agent.

14. A solubilizer which consists of a water soluble amphiphilic polyelectrolyte obtained by copolymerizing (meth)acrylamido alkylsulfonic acid and/or its salt and (meth)acrylate, or by neutralizing the obtained copolymer with an alkali agent.

15. A solubilizer which consists of a water soluble cross-linked amphiphilic polyelectrolyte obtained by copolymerizing (meth)acrylamido alkylsulfonic acid and/or its salt, (meth)acrylate and a cross-linking agent, or by neutralizing the obtained cross -linked copolymer with an alkali agent.

16. The solubilizer of claim 12 or 13 wherein at least 15 wt% of the total of said water soluble amphiphilic polyelectrolyte is (meth)acrylamido alkylsulfonic acid units.

17. The solubilizer of claim 12 or 13 wherein at least 40 wt% of the total of said water soluble amphiphilic polyelectrolyte is N-substituted (meth) acrylamide units.

18. The solubilizer of claim 14 or 15 wherein at least 60 wt% of the total of said water soluble amphiphilic polyelectrolyte is (meth)acrylamido alkylsulfonic acid units.

19. The solubilizer of claim 14 or 15 wherein at least 1 wt% of the total of said water soluble amphiphilic polyelectrolyte is (meth)acrylate units.

20. A solubilized composition comprising the solubilizer of claim 12, 13 14 or 15.

21. The solubilized composition of claim 20 wherein the blend ratio of said solubilizer is 0.01-20 wt%.

22. A solubilized cosmetic comprising the solubilizer of claim 12, 13, 14 or 15.
